(19) Europäisches Patentamt
European Patent Office
Office européen des brevets

(11) Numéro de publication: 0 319 991 B1

(12) **FASCICULE DE BREVET EUROPEEN**

(45) Date de publication de fascicule du brevet: **19.02.92** (51) Int. Cl.$^5$: **A61K 7/06**

(21) Numéro de dépôt: **88120602.3**

(22) Date de dépôt: **09.12.88**

# (54) Association de dérivés de pyrimidine et d'agents antibactériens pour induire et stimuler la croissance des cheveux et diminuer leur chute.

(30) Priorité: **10.12.87 LU 87070**

(43) Date de publication de la demande:
**14.06.89 Bulletin 89/24**

(45) Mention de la délivrance du brevet:
**19.02.92 Bulletin 92/08**

(84) Etats contractants désignés:
**AT BE CH DE FR GB IT LI NL SE**

(56) Documents cités:
**FR-A- 2 598 420**
**GB-A- 2 175 303**

**PATENT ABSTRACTS OF JAPAN, vol. 11, no. 115 (C-415)[2562], 10 avril 1987; page 145 C 415**

(73) Titulaire: **L'OREAL**
**14, Rue Royale**
**F-75008 Paris(FR)**

(72) Inventeur: **Grollier, Jean Francois**
**16 Bis Boulevard Morland**
**F-75004 Paris(FR)**
Inventeur: **Rosenbaum, Georges**
**2, rue J.H. Mansart**
**F-92600 Asnières(FR)**

(74) Mandataire: **Casalonga, Axel et al**
**BUREAU D.A. CASALONGA - JOSSE Morassistrasse 8**
**W-8000 München 5(DE)**

EP 0 319 991 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention est relative à l'association de dérivés de pyrimidine et de certains agents antibactériens pour une utilisation simultanée, successive ou décalée dans le temps en vue d'induire et de stimuler la croissance des cheveux et diminuer leur chute.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle pilaire au cours duquel le cheveu se forme, croît et tombe avant d'être remplacé par un nouveau cheveu qui apparaît dans le même follicule.

On observe, au cours d'un cycle pilaire, successivement trois phases, à savoir : la phase anagène, la phase catagène et la phase télogène.

Au cours de la première phase, dite anagène, le cheveu passe par une période de croissance active associée avec une intense activité métabolique au niveau du bulbe.

La deuxième phase, dite catagène, est transitoire et elle est marquée par un ralentissement des activités mitotiques. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber, poussé par un cheveu anagène naissant.

Ce processus de renouvellement physique permanent subit une évolution naturelle au cours du vieillissement, les cheveux deviennent plus fins, leurs cycles plus courts.

L'alopécie survient lorsque ce processus de renouvellement physique est accéléré ou perturbé, c'est-à-dire que les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté. Ce phénomène peut conduire à la calvitie.

Le cycle pilaire est tributaire de nombreux facteurs pouvant entraîner une alopécie plus ou moins prononcée. Parmi ces facteurs, ou peut citer les facteurs alimentaires, endocriniens, nerveux; etc.

On détermine la variation des cheveux dans les différentes phases grâce au trichogramme et en particulier au photo-trichogramme, qui permet, notamment, de déterminer le nombre de cheveux en phase anagène par rapport au nombre de cheveux en phase télogène.

On recherche depuis de nombreuses années dans l'industrie cosmétique ou pharmaceutique, des compositions permettant de supprimer ou de réduire l'effet de l'alopécie, et notamment d'induire ou de stimuler la croissance des cheveux ou de diminuer leur chute.

Dans cette optique, on a déjà proposé des composés tels que l'amino-6 dihydro-1,2 hydroxy-1 imino-2 pipéridino-4 pyrimidine et ses dérivés, en particulier le "Minoxidil". De tels composés sont décrits, notamment, dans le brevet US 4.139.619.

La demanderesse a découvert qu'en associant un agent antibactérien choisi parmi les antibiotiques de la famille des macrolides, des pyranosides, ainsi que la tétracycline avec un dérivé de pyrimidine, il était possible de façon surprenante d'obtenir une plus grande efficacité qu'avec des compositions antérieurement connues ne renfermant que des dérivés de pyrimidine.

Ce résultat est particulièrement surprenant lorsqu'on sait que les antibiotiques en question ne sont pas connus pour avoir un effet au niveau de la croissance des cheveux ou au niveau de leur action sur le freinage de la chute des cheveux.

Cette efficacité se traduit, notamment, par une activité supérieure à une même concentration, déterminée en particulier par un phototrichogramme, par une action plus rapide ou encore par une utilisation du dérivé de pyrimidine à une concentration plus faible.

Cet effet a été constaté, non seulement pour des compositions contenant simultanément le dérivé de pyrimidine et les antibiotiques cités ci-dessus, mais également lorsqu'ils étaient utilisés dans des étapes séparées.

L'invention a donc pour objet une association de dérivés de pyrimidine et d'agents antibactériens choisis parmi les antibiotiques de la famille des macrolides, des pyranosides et des tétracyclines.

Un autre objet de l'invention est constitué par les compositions contenant ces agents.

D'autres objets de l'invention apparaîtront à la lecture de la description et des exemples qui suivent.

L'association conforme à l'invention est essentiellement caractérisée par le fait qu'elle comprend :

a/ un composant (A) contenant dans un milieu physiologiquement acceptable un agent antibactérien choisi parmi les macrolides, les pyranosides et les tétracyclines;

b/ un composant (B) contenant dans un milieu physiologiquement acceptable un dérivé de pyrimidine répondant à la formule :

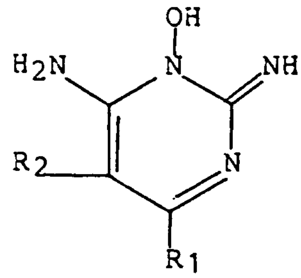

(I)

dans laquelle $R_1$ désigne un groupement

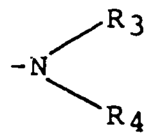

dans lequel $R_3$ et $R_4$ peuvent être choisis parmi l'hydrogène, un groupement alkyle, alcényle, alkylaryle ou cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition et étant destinés à être utilisés séparément, soit simultanément, soit de façon successive ou décalée dans le temps, en vue d'induire et de stimuler la croissance des cheveux et diminuer leur chute.

Pour les composés de formule (I), le groupement alkyle ou alcoxy désigne de préférence un groupement ayant 1 à 4 atomes de carbone; le groupement alcényle désigne de préférence un groupement ayant 2 à 5 atomes de carbone; le groupement aryle désigne de préférence un groupement phényle et le groupement cycloalkyle désigne de préférence un groupement ayant 4 à 6 atomes de carbone.

Les composés particulièrement préférés de formule (I) sont choisis parmi les composés dans lesquels $R_2$ désigne hydrogène et $R_1$ désigne un groupement :

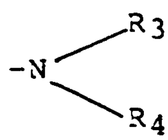

dans lequel $R_3$ et $R_4$ forment un cycle pipéridinyle, ainsi que leurs sels tels que, par exemple, le sulfate.

Parmi ces composés, un composé particulièrement préféré est constitué par l'amino-6 dihydro-1,2 hydroxy-1 imino-2 pipéridino-4 pyrimidine, encore appelé "Minoxidil".

Les agents antibactériens de la famille des macrolides sont plus particulièrement choisis parmi l'érythromycine et ses dérivés, et notamment l'érythromycine elle-même ainsi que l'estolate, l'éthylcarbonate, l'éthylsuccinate, le glucoheptonate, le lactobionate, le propionyl laurylsulfate, le propionate, le stéarate, le linoléate, et les esters mono-éniques, bi- ou tri-éniques d'érythromycine, ainsi que les esters d'acide rétinoïque all-trans et 13-cis d'érythromycine A, et leurs sels pharmaceutiquement acceptables tels que décrits plus particulièrement dans la demande de brevet français n° 86.06528 de la demanderesse, et dans le brevet français 2.585.000.

Les esters rétinoïques en position 2′ d'érythromycine sont représentés plus particulièrement par la formule :

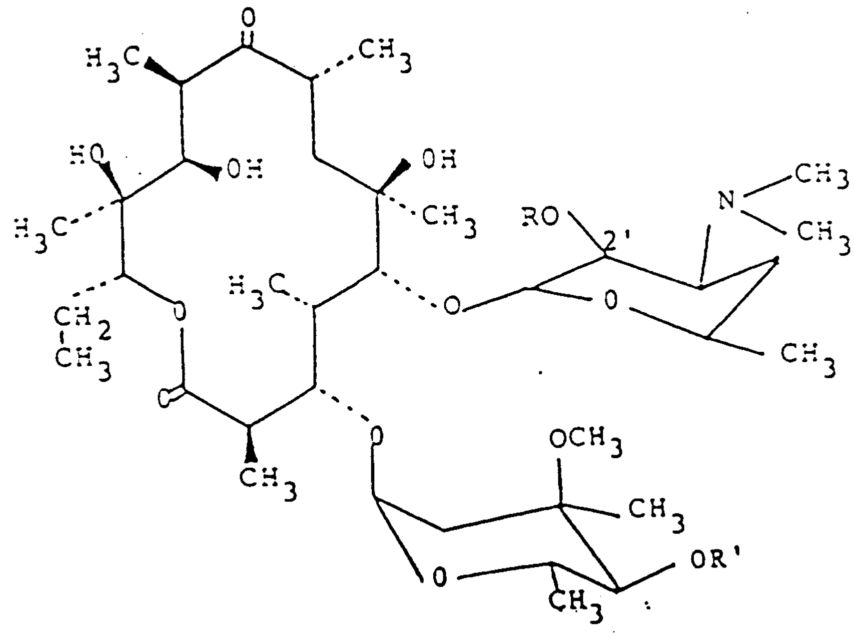

(II)

dans laquelle R représente le radical rétinoyle all-trans ou le radical rétinoyle 13-cis, et R′ désigne H. Le radical rétinoyle ayant pour formule :

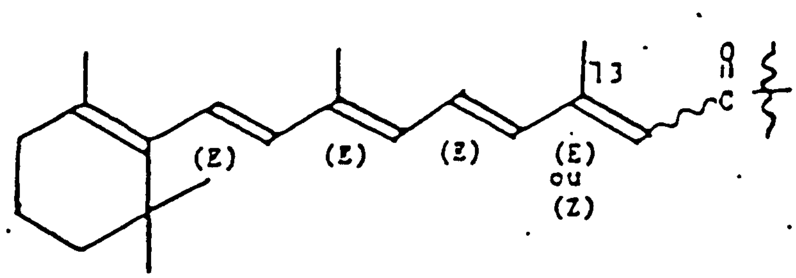

(III)

Ces différents esters rétinoïques peuvent être préparés suivant différents procédés d'estérification et de préférence dans un milieu solvant organique anhydre, en particulier dans le tétrahydrofuranne seul ou en mélange avec un autre solvant organique comme la pyridine, en faisant réagir un excès d'anhydride carbonique mixte des acides rétinoïques all-trans ou 13-cis (préparé in situ, par exemple à partir de chloroformiate d'éthyle et d'acide all-trans ou 13-cis) avec l'érythromycine A, sous forme de base, en présence d'une base organique ou minérale comme la pyridine et/ou l'hydrogénocarbonate de sodium.

D'autres dérivés d'érythromycine A sont ceux représentés par la formule (II) ci-dessus, dans laquelle :

R ou R′ représente un radical acyle linéaire en $C_{18}$ bi- ou tri-énique, de configuration stéréochimique all-cis (Z) et R′ ou R restant représente un atome d'hydrogène.

Selon une forme de réalisation préférée, R ou R′ représente dans ces composés les radicaux suivants :

Z-9, Z-12-octadécadiénoyle ou linoléoyle

Z-9, Z-12, Z-15-octadécatriénoyle ou α-linolénoyle, et

Z-6, Z-9 Z-12-octadécatriénoyle ou α-linolénoyle.

On peut citer plus particulièrement 1′0-linoleoyl-2′ érythromycine A, 1′0-linoleoyl-4″ érythromycine A et 1′0-αlinoleoyl-4″ érythromycine A.

Les agents antibactériens de la famille des macrolides sont également choisis parmi la spiramycine et ses dérivés tels que par exemple le di- ou le triacétate, l'oléandomycine ou son chlorhydrate, ainsi que la Roxytromycine, la Josamycine et leurs dérivés.

Les pyranosides sont choisis plus particulièrement parmi la clindamycine et ses dérivés, tels que le chlorhydrate, le palmitate et le phosphate, la lincomycine et ses dérivés, tels que le chlorhydrate. D'autres dérivés de clindamycine ou de lincomycine sont les rétinoates de lincomycine et de clindamycine et leurs sels pharmaceutiquement acceptables tels que décrits plus particulièrement dans la demande de brevet français n° 86.06528 de la demanderesse, représentés par les formules :

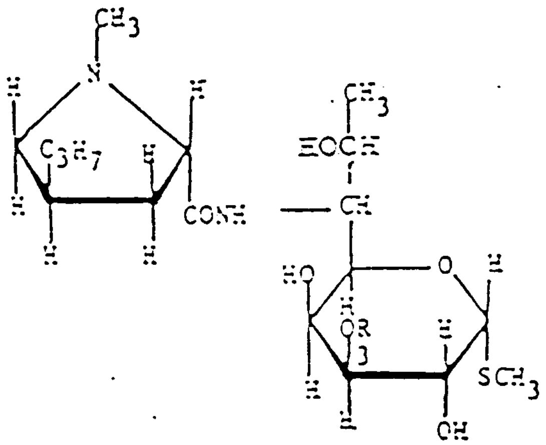

(IV)

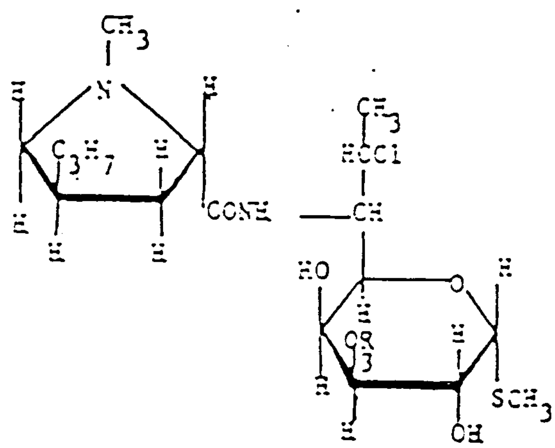

(V)

dans lesquelles R représente le radical rétinoyle de formule (III) di-dessus.

Les esters sont préparés comme indiqué ci-dessus pour les rétinoates d'érythromycine.

Un autre procédé de préparation consiste à utiliser pour la lincomycine et la clindamycine des imidazolides d'acides rétinoïques dans un solvant anhydre comme la N,N-diméthylformamide, en présence d'une base comme le tertiobutylate de sodium ou de potassium. Selon ce dernier procédé, l'ester, en position 7 de la lincomycine est obtenu en majorité avec des esters en positions 2, 3 et 4. On obtient de la même façon un mélange de monoesters en positions 2, 3 et 4 de la clindamycine.

Les dérivés de tétracycline sont plus particulièrement choisis parmi la tétracycline et ses dérivés, tels que le chlorhydrate, le N-(n-dodécyl) sulfamate, le guaïcolglycolate, le guaïcolsulfonate, le laurylsulfate, le métaphosphate, le complexe tétracyclinephosphate, le complexe de tartrate, le phénoxyméthylpénicillinate, le timolsulfonate, le triméthoxybenzoate trihydraté.

Les composants (A) et (B) ainsi que la composition les contenant dans une forme de réalisation, peuvent se présenter sous des formes diverses habituellement utilisées pour une application topique, et plus particulièrement sous forme de lotion éventuellement épaissie, de gel, d'émulsion, de mousse éventuellement conditionnée en aérosol, de spray, de stick.

Le milieu physiologiquement acceptable peut être constitué par de l'eau, un mélange d'eau et d'un solvant ou un mélange de solvants, les solvants étant choisis parmi les solvants organiques, acceptables sur le plan cosmétique ou pharmaceutique, et choisis plus particulièrement parmi les alcools inférieurs en $C_1$-$C_4$ comme l'alcool éthylique, l'alcool isopropylique, l'alcool tertiobutylique, les alkylèneglycols, les alkyléthers d'alkylèneglycol, de dialkylèneglycol, tels que plus particulièrement le monoéthyléther d'éthylèneglycol, le monométhyléther de propylèneglycol et le monoéthyléther de diéthylèneglycol.

Les solvants sont présents de préférence dans des proportions comprises entre 1 et 80% en poids par rapport au poids total de la composition.

Une forme de réalisation préférée consiste à utiliser un milieu anhydre, c'est-à-dire comprenant moins de 1% d'eau et constituée d'un ou plusieurs solvant(s) tels que définis ci-dessus.

Les milieux physiologiquement acceptables peuvent être épaissis à l'aide d'agents épaississants habituellement utilisés en cosmétique ou pharmacie et on peut utiliser, plus particulièrement, les hétérobiopolysaccharides tels que la gomme de xanthane, les scléroglucanes, les dérivés de cellulose, les polymères acryliques réticulés ou non.

Les épaississants sont présents de préférence dans des proportions comprises entre 0,1 à 5% en poids, et en particulier entre 0,4 et 3% en poids par rapport au poids total de chacun des composants lorsqu'ils sont utilisés de façon séparée, ou par rapport au poids total de la composition contenant les composants (A) et (B).

Les compositions constituées, soit par les composants (A) et (B) ou par la composition contenant simultanément les deux composants, peuvent également contenir tous autres adjuvants habituellement utilisés dans les compositions destinées à une application topique à utilisation cosmétique ou pharmaceutique, et plus particulièrement des agents conservateurs, des agents complexants, des colorants, des agents alcalinisants ou acidifidants, des agents tensio-actifs, anioniques, cationiques, non ioniques, amphotères, ou leurs mélanges, des polymères anioniques, cationiques, non ioniques ou amphotères ainsi que leurs

mélanges.

Le pH de ces compositions peut varier entre 4 et 9.

Les agents antibactériens utilisés conformément à l'invention peuvent être présents dans le composant (A) dans des proportions comprises entre 0,01 et 10% en poids par rapport au poids total du composant (A) et en particulier entre 0,1 et 5% en poids et plus particulièrement encore entre 0,3 et 5% en poids.

Les dérivés de pyrimidine de formule (I) sont utilisés dans le composant (B), de préférence dans des proportions comprises entre 0,05 et 10% en poids, et de préférence entre 0,1 et 5% en poids, et en particulier entre 0,5 et 3% en poids par rapport au poids total du composant (B).

Dans le composant (B), les dérivés de pyrimidine de formule (I) peuvent être présents, soit sous forme dissoute dans les milieux physiologiquement acceptables, ou alors en totalité ou partiellement en suspension dans ces milieux, en particulier sous forme de particules ayant une granulométrie inférieure à 80 µm et de préférence inférieure à 20 µm, et en particulier inférieure à 5 µm.

Une forme de réalisation de l'invention consiste à utiliser l'association telle que définie ci-dessus sous forme d'une composition unique contenant les composants (A) et (B). Dans ce cas, la concentration en agents antibactériens est de préférence comprise entre 0,1 et 3% en poids par rapport au poids total de la composition et celle du dérivé de pyrimidine de formule (I) est de préférence comprise entre 0,5 et 3% en poids par rapport au poids total de la composition.

Une forme particulièrement préférée de l'invention consiste à appliquer simultanément les composants (A) et (B) conservés dans des dispositifs séparés et renfermant de préférence les substances actives, à savoir l'agent antibactérien et le dérivé de pyrimidine dans un mélange de solvants organiques comprenant par exemple de l'alcool éthylique et du propylèneglycol ou un mélange de solvant et d'eau tel qu'un mélange d'eau et d'alcool éthylique et de propylèneglcol.

Une autre forme de réalisation consiste à appliquer dans un premier temps le composant (A) contenant l'antibiotique tel que défini ci-dessus, puis à appliquer le composant (B) contenant le dérivé de pyrimidine de formule (I) ou inversement, les applications pouvant s'effectuer en succession immédiate ou décalée dans le temps.

L'association conforme à l'invention peut être conditionnée dans ces cas, en particulier dans un dispositif à plusieurs compartiments encore appelé "kit" ou nécessaire, le premier compartiment contenant le composant (A) avec l'agent antibactérien, et le second compartiment contenant le composant (B) à base du dérivé de pyrimidine de formule (I). Ce dispositif peut être équipé d'un moyen de mélange des composants (A) et (B) tout juste au moment de l'application.

Le traitement pour induire et stimuler la croissance des cheveux et diminuer leur chute consiste principalement à appliquer sur les zones alopéciques du cuir chevelu d'un individu, l'association telle que définie ci-dessus, soit au moyen d'une composition unique, soit par application des composants (A) et (B) ou (B) et (A) de façon successive et décalée dans le temps.

Le mode d'application préféré consiste à appliquer 1 à 2 g de la composition ou de chacun des composants (A) et (B) sur la zone alopécique à une fréquence de une à deux applications par jour pendant 1 à 7 jours par semaine et ceci pendant une durée de 1 à 6 mois.

Le procédé de traitement pour induire et stimuler la croissance des cheveux ou diminuer leur chute, qui constitue un des objets de l'invention, présente les caractéristiques d'un traitement de thérapeutique dans la mesure où l'association conforme à l'invention a une activité thérapeutique au niveau des mécanismes biologiques du cycle pilaire et restaure le dysfonctionnement de celui-ci.

L'invention a donc également pour objet l'association telle que définie ci-dessus pour son application comme médicament dans le traitement de la chute des cheveux.

Un autre objet de l'invention est constitué par l'utilisation de l'association définie ci-dessus pour la préparation d'un médicament ayant pour effet d'induire ou de stimuler la croissance des cheveux et de freiner leur chute.

Le procédé conforme à l'invention présente enfin les caractéristiques d'un procédé cosmétique dans la mesure où il permet d'améliorer l'esthétique des cheveux ou du cuir chevelu.

Les exemples suivants sont destinés à illustrer l'invention sans pour autant présenter un caractère limitatif.

## EXEMPLE 1

On conditionne en kit deux compositions (A) et (B) renfermant respectivement :

| COMPOSITION (A) : | |
|---|---|
| - Erythromycine<br>- Alcool éthylique<br>- Propylèneglycol | 0,5 g<br>95,0 g<br>qsp 100,0 g |
| COMPOSITION (B) : | |
| - Minoxidil<br>- Propylène glycol<br>- Alcool éthylique<br>- Eau | 2,0 g<br>20,0 g<br>50,0 g<br>qsp 100,0 g |

On applique successivement à la suite l'une de l'autre les compositions (A) puis (B) sur les parties alopéciques du cuir chevelu, à raison environ de 5 mg par $cm^2$. Le traitement journalier est poursuivi sur 2 mois. On constate, à l'examen du trichogramme une augmentation significative des cheveux en phase anagène.

EXEMPLE 2

On conditionne en kit deux compositions (A) et (B) renfermant respectivement :

| COMPOSITION (A) : | |
|---|---|
| - Estolate d'érythromycine (ou érythromycine 2′ propionate dodécylsulfate)<br>- Propylèneglycol<br>- Alcool éthylique<br>- Eau | 4,0 g<br>20,0 g<br>50,0 g<br>qsp 100,0 g |
| COMPOSITION (B) : | |
| - Minoxidil<br>- Alcool éthylique<br>- Propylèneglycol | 2,0 g<br>95,0 g<br>qsp 100,0 g |

On applique le mélange extemporané des deux compositions (A) et (B).
On note des résultats similaires à ceux de l'exemple 1.

EXEMPLE 3

On conditionne en kit deux compositions (A) et (B) renfermant respectivement :

| COMPOSITION (A) : | |
|---|---|
| - Ethylsuccinate d'érythromycine (ou érythromycine 2′ éthyl-butanedioate)<br>- Alcool éthylique<br>- Propylèneglycol | 1,0 g<br>95,0 g<br>qsp 100,0 g |
| COMPOSITION (B) : | |
| - Minoxidil<br>- Alcool éthylique<br>- Propylèneglycol | 2,0 g<br>95,0 g<br>qsp 100,0 g |

On applique successivement à quelques heures d'intervalle les compositions (B) puis (A).
On note des résultats similaires à ceux de l'exemple 1.

EXEMPLE 4

On prépare la composition suivante :

| | |
|---|---|
| - Erythromycine | 0,5 g |
| - Minoxidil | 1,0 g |
| - Alcool éthylique | 95,0 g |
| - Propylèneglycol | qsp 100,0 g |

Appliquée sur les cheveux à raison de 7 mg par $cm^2$, 1 fois par jour pendant 2 mois, on note des résultats similaires à ceux de l'exemple 1.

EXEMPLE 5

On prépare la composition suivante :

| | |
|---|---|
| - Erythromycine | 1,0 g |
| - Minoxidil | 2,0 g |
| - Alcool éthylique | 50,0 g |
| - Propylèneglycol | 20,0 g |
| - Eau | qsp 100,0 g |

Appliquée sur les cheveux à raison de 4 mg par $cm^2$, 1 fois par jour pendant 2 mois, on note des résultats similaires à ceux de l'exemple 1.

EXEMPLE 6

On prépare une lotion de composition suivante :

| | |
|---|---|
| - Spiramycine | 1,0 g |
| - Minoxidil, | 1,0 g |
| - Propylèneglycol | 20,0 g |
| - Alcool éthylique | 50,0 g |
| - Eau | qsp 100,0 g |

Appliquée sur les cheveux à raison de 7 mg par $cm^2$, 1 fois par jour pendant 2 mois, on note des résultats similaires à ceux de l'exemple 1.

EXEMPLE 7

On prépare une lotion de composition suivante :

| | |
|---|---|
| - Tétracycline base | 0,5 g |
| - Minoxidil | 1,5 g |
| - Alcool éthylique | 50,0 g |
| - Propylèneglycol | 20,0 g |
| - Eau | qsp 100,0 g |

Appliquée sur les cheveux à raison de 6 mg par $cm^2$, 1 fois par jour pendant 2 mois, on note des résultats similaires à ceux de l'exemple 1.

EXEMPLE 8

On conditionne en kit deux compositions (A) et (B) renfermant respectivement :

| Composition (A) : | |
|---|---|
| - Oléandomycine<br>- Propylèneglycol<br>- Alcool éthylique<br>- Eau | 0,5 g<br>20,0 g<br>50,0 g<br>qsp 100,0 g |
| Composition (B) : | |
| - Minoxidil<br>- Propylèneglycol<br>- Alcool éthylique<br>- Eau | 2,0 g<br>20,0 g<br>50,0 g<br>qsp 100,0 g |

On applique les compositions en succession décalée dans le temps, (A) le matin, (B) le soir.

On note des résultats similaires à ceux de l'exemple 1.

EXEMPLE 9

On conditionne en kit deux compositions (A) et (B) renfermant respectivement :

| Composition (A) : | |
|---|---|
| - Tétracycline chlorhydrate<br>- Propylèneglycol<br>- Alcool éthylique<br>- Eau | 1,0 g<br>20,0 g<br>50,0 g<br>qsp 100,0 g |
| Composition (B) : | |
| - Minoxidil de diamètre particulaire moyen inférieur à 2 microns<br>- Acide polyacrylique réticulé PM : 3 millions, vendu sous la dénomination "CARBOPOL® 934" par la Société GOODRICH<br>- Propylèneglycol<br>- Amino-2 méthyl-2 propanol-1 qs pH = 7<br>- Conservateur qs<br>- Eau | 1,25 g<br><br>1,0 g<br><br>4,5 g<br><br><br>qsp 100,0 g |

On applique les compositions en succession décalée dans le temps, en alternance journalière : premier jour, la composition (A); second jour, la composition (B).

On note des résultats similaires à ceux de l'exemple 1.

EXEMPLE 10

On prépare la composition suivante :

| | |
|---|---|
| - Lincomycine | 0,6 g |
| - Minoxidil | 0,5 g |
| - Alcool éthylique | 95,0 g |
| - Propylèneglycol | qsp 100,0 g |

Appliqué sur les cheveux à raison de 8 mg par $cm^2$, 1 fois par jour pendant 2 mois, on note des résultats similaires à ceux de l'exemple 1.

EXEMPLE 11

On conditionne en kit deux compositions (A) et (B) renfermant respectivement :

| Composition (A) : | |
|---|---|
| - Clindamycine<br>- Alcool éthylique<br>- Eau | 0,3 g<br>50,0 g<br>qsp 100,0 g |
| Composition (B) : | |
| - Minoxidil<br>- Alcool éthylique<br>- Propylèneglycol | 1,0 g<br>95,0 g<br>qsp 100,0 g |

On applique les deux compositions (A) puis (B) en succession immédiate.
On note des résultats similaires à ceux de l'exemple 1.

EXEMPLE 12

On conditionne en kit deux compositions (A) et (B) renfermant respectivement :

Composition (A) :

| | |
|---|---|
| - Erythromycine | 5,0 g |
| - Butylhydroxytoluène | 0,125 g |
| { Propylèneglycol (5 g)<br>{ Alcool éthylique (95 g) | qsp 100,0 g |

Composition (B) :

| | |
|---|---|
| - Minoxidil | 2,5 g |
| - Butylhydroxytoluène | 0,125 g |
| { Propylèneglycol (5 g)<br>{ Alcool éthylique (95 g) | qsp 100,0 g |

On applique le mélange extemporané des deux compositions :
(A) : 75% poids, et
(B) : 25% poids.

EXEMPLE 13

On prépare la composition suivante :

| | | |
|---|---|---|
| - Minoxidil | | 0,625 g |
| - Erythromycine | | 3,0 g |
| - Butylhydroxytoluène | | 0,125 g |
| Propylèneglycol (5 g) / Alcool éthylique (95 g) | qsp | 100,0 g |

On applique cette composition une fois par jour sur les zones alopéciques du cuir chevelu pendant trois mois.

**Revendications**

1. Association destinée à induire et à stimuler la croissance des cheveux et à diminuer leur chute, caractérisée par le fait qu'elle comprend :
   a/ un composant (A) contenant dans un milieu physiologiquement acceptable, au moins un agent antibactérien choisi parmi les antibiotiques de la famille des macrolides, des pyranosides ainsi que des tétracyclines; et
   b/ un composant (B) contenant, dans un milieu physiologiquement acceptable, au moins un dérivé de pyrimidine répondant à la formule :

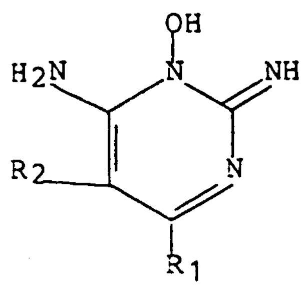

(I)

dans laquelle $R_1$ désigne un groupement

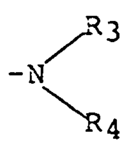

dans lequel $R_3$ et $R_4$ peuvent être choisis parmi l'hydrogène, un groupement alkyle, alcényle, alkylaryle ou cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyl, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

2. Association selon la revendication 1, caractérisée par le fait que les agents antibactériens de la famille des macrolides sont choisis parmi l'érythromycine, la Spiramycine, l'Oléandomycine; la Roxytromycine,

la Josamycine et leurs dérivés et que les pyranosides sont choisis parmi la lincomycine, la clindamycine et leurs dérivés.

3. Association selon la revendication 2, caractérisée par le fait que les dérivés d'érythromycine sont choisis parmi l'estolate, l'éthylcarbonate, l'éthylsuccinate, le glucoheptonate, le lactobionate, le propionyl laurylsulfate, le propionate, le stéarate, le linoléate, les esters mono-éniques, bi- ou tri-éniques d'érythromycine, les esters d'acide rétinoïque all-trans et 13-cis d'érythromycine A, ainsi que 1′0-linoleoyl-2′ érythromycine A, 1′0-linoleoyl-4″ érythromycine A et 1′0-α-linoleoyl-4″ érythromycine A, que les dérivés de spiramycine sont choisis parmi le di- ou triacétate de spiramycine, que le dérivé de clindamycine est le chlorhydrate.

4. Association selon l'une quelconque des revendications 1 à 3, caractérisée par le fait que les dérivés de lincomycine sont choisis parmi la lincomycine et le chlorhydrate de lincomycine; que les dérivés de clindamycine sont choisis parmi la clindamycine, et les chlorhydrate, palmitate et phosphate de clindamycine, ainsi que les dérivés d'acide rétinoïque de lincomycine et de clindamycine.

5. Association selon l'une quelconque des revendications 1 à 4, caractérisée par le fait que les dérivés de tétracycline sont choisis parmi la tétracycline, le chlorhydrate, le N-(n-dodécyl) sulfamate, le guaïcolglycolate, le guaïcolsulfonate, le laurylsulfate, le métaphosphate, le complexe tétracyclinephosphate, le complexe de tartrate, le phénoxyméthylpénicillinate, le timolsulfonate, le triméthoxybenzoate trihydraté de tétracycline.

6. Association selon la revendication 5, caractérisée par le fait que le dérivé de pyrimidine est 1′amino-6 dihydro-1,2 hydroxy-1 imino-2 pipéridino-4 pyrimidine ou Minoxidil.

7. Association selon l'une quelconque des revendications 1 à 6, caractérisée par le fait que l'agent antibactérien de la famille des antibiotiques est présent dans le composant (A) dans des proportions comprises entre 0,01 et 10% en poids par rapport au poids total du composant (A) et en particulier entre 0,1 et 5% en poids, plus particulièrement encore entre 0,3 et 5% et que le dérivé de pyrimidine de formule (I) est présent dans le composant (B) dans des proportions comprises entre 0,05 et 10% en poids et de préférence entre 0,1 et 5% en poids, et en particulier entre 0,5 et 3% en poids.

8. Association selon l'une quelconque des revendications 1 à 7, caractérisée par le fait que l'association est présente dans une composition unique, dans laquelle l'agent antibactérien est présent dans des proportions comprises entre 0,1 et 3% en poids et que le dérivé de pyrimidine de formule (I) est présent dans une proportion comprise entre 0,5 et 3% en poids par rapport au poids total de la composition.

9. Association selon l'une quelconque des revendications 1 à 8, caractérisée par le fait que le milieu physiologiquement acceptable pour les composants (A) et (B) est un milieu constitué par de l'eau ou un mélange d'eau et d'un ou plusieurs solvants organiques ou par un mélange de solvants organiques acceptable en cosmétique ou en pharmacie.

10. Association selon l'une quelconque des revendications 1 à 9, caractérisée par le fait que l'un au moins des composants (A) ou (B) contient un solvant choisi parmi les alcools inférieurs en $C_1$-$C_4$, les alkylèneglycols, les alkyléthers de mono- et dialkylèneglycol.

11. Association selon l'une quelconque des revendications 1 à 10, caractérisée par le fait que l'un au moins des composants (A) ou (B) est épaissi au moyen d'agents épaississants ou gélifiants.

12. Association selon l'une quelconque des revendications 1 à 11, caractérisée par le fait que l'un au moins des composants (A) ou (B) contient également un adjuvant cosmétiquement ou pharmaceutiquement acceptable choisi parmi les agents conservateurs, les agents complexants, les colorants, les agents alcalinisants ou acidifiants, les agents tensio-actifs, anioniques, cationiques, non ioniques, amphotères ou leurs mélanges, les polymères anioniques, cationiques, non ioniques ou amphotères ainsi que leurs mélanges.

13. Procédé de traitement cosmétique des cheveux ou du cuir chevelu, caractérisé par le fait que l'on

applique les composants (A) et (B) de l'association telle que définie dans l'une quelconque des revendications 1 à 12, soit au moyen d'un composition unique, soit de façon séparée, soit simultanément, soit de façon successive ou décalée dans le temps.

**14.** Dispositif à plusieurs compartiments ou "kit", caractérisé par le fait qu'il comprend dans un premier compartiment le composant (A) tel que défini dans l'une quelconque des revendications 1 à 12, et dans un second compartiment le composant (B) tel que défini dans l'une quelconque des revendications 1 à 12.

**15.** Association selon l'une quelconque des revendications 1 à 12, pour son application comme médicament dans le traitement thérapeutique de la chute des cheveux.

**16.** Utilisation de l'association selon l'une quelconque des revendications 1 à 12, pour la préparation d'un médicament destiné au traitement thérapeutique de la chute des cheveux.

## Claims

**1.** Combination intended to induce and to stimulate the growth of hair and to reduce their loss, characterised in that it comprises:

a) a component (A) containing in a physiologically acceptable medium, at least one antibacterial agent chosen from antibiotics of the macrolide, pyranoside as well as tetracycline families; and

b) one component (B) containing in a physiologically acceptable medium, at least one pyrimidine derivative of the formula:

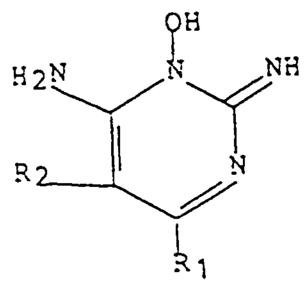

(I)

in which $R_1$ denotes a group

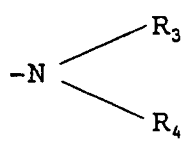

in which $R_3$ and $R_4$ may be chosen from hydrogen, an alkyl, an alkenyl, an alkylaryl or a cycloalkyl group, $R_3$ and $R_4$ may also form a heterocycle with the nitrogen atom to which they are attached, chosen from the aziridinyl, azetidinyl, pyrrolidinyl, piperidinyl, hexahydroazepinyl, heptamethyleneimine, octamethyleneimine, morpholine and 4-alkylpiperazidinyl groups, it being possible for the heterocyclic groups to be substituted on the carbon atoms by one to three lower alkyl, hydroxy or alkoxy groups; the group $R_2$ is chosen from a hydrogen atom, an alkyl, an alkenyl, an alkylalkoxy, a cycloalkyl, an aryl, an alkylaryl, an arylalkyl, an alkylarylalkyl, an alkoxyarylalkyl or a haloarylalkyl group, as well as the addition salts of physiologically acceptable acids, the components (A) and (B) being part of the same composition or being intended to be used on the scalp or on the hair separately or simultaneously either successively or spaced out over time.

**2.** Combination according to Claim 1, characterised in that the antibacterial agents of the macrolide family are chosen from erythromycin, spiramycin, oleandomycin, roxytromycin, josamycin and their derivatives and in that the pyranosides are chosen from lincomycin, clindamycin and their derivatives.

**3.** Combination according to Claim 2, characterised in that the erythromycin derivatives are chosen from

erythromycin estolate, ethyl carbonate, ethyl succinate, glucoheptonate, lactobionate, propionyl lauryl sulphate, propionate, stearate, linoleate, the mono-, bi- or trienic esters of erythromycin, the all-trans- and 13-cis-retinoic acid esters of erythromycin A, as well as 2'-O-linoleoylerythromycin A, 4"-O-linoleoylerythromycin A and 4"-O-α-linoleoylerythromycin A, in that the spiramycin derivatives are chosen from spiramycin di- or triacetate, in that the clindamycin derivative is the hydrochloride.

**4.** Combination according to any one of Claims 1 to 3, characterised in that the lincomycin derivatives are chosen from lincomycin and lincomycin hydrochloride; in that the clindamycin derivatives are chosen from clindamycin and clindamycin hydrochloride, palmitate and phosphate, as well as the lincomycin and clindamycin retinoic acid derivatives.

**5.** Combination according to any one of Claims 1 to 4, characterised in that the tetracycline derivatives are chosen from tetracycline, tetracycline hydrochloride, N-(n-dodecyl)sulphamate, guaicolglycolate, guaicolsulphonate, lauryl sulphate, metaphosphate, tetracyclinephosphate complex, tartrate complex, phenoxymethylpenicillinate, timolsulphonate, trimethoxybenzoate trihydrate.

**6.** Combination according to Claim 5, characterised in that the pyrimidine derivative is 6-amino-1,2-dihydro-1-hydroxy-2-amino-4-piperidinopyrimidine or minoxidil.

**7.** Combination according to any one of Claims 1 to 6, characterised in that the antibacterial agent of the antibiotic family is present in component (A) in proportions of between 0.01 and 10% by weight relative to the total weight of component (A) and in particular between 0.1 and 5% by weight, still more particularly between 0.3 and 5% and in that the pyrimidine derivative of formula (I) is present in component (B) in proportions of between 0.05 and 10% by weight and preferably between 0.1 and 5% by weight, and in particular between 0.5 and 3% by weight.

**8.** Combination according to any one of Claims 1 to 7, characterised in that the combination is present in a single composition in which the antibacterial agent is present in proportions of between 0.1 and 3% by weight and in that the pyrimidine derivative of formula (I) is present in a proportion of between 0.5 and 3% by weight relative to the total weight of the composition.

**9.** Combination according to any one of Claims 1 to 8, characterised in that the physiologically acceptable medium for components (A) and (B) is a medium consisting of water or a mixture of water and one or more organic solvents or of a mixture of cosmetically or pharmaceutically acceptable organic solvents.

**10.** Combination according to any one of Claims 1 to 9, characterised in that at least one of the components (A) or (B) contains a solvent chosen from $C_1$-$C_4$ lower alcohols, alkylene glycols and mono- and dialkylene glycol alkylethers.

**11.** Combination according to any one of Claims 1 to 10, characterised in that at least one of the components (A) or (B) is thickened by means of thickening or jellied agents.

**12.** Combination according to any one of Claims 1 to 11, characterised in that at least one of the components (A) or (B) also contains a cosmetically or pharmaceutically acceptable adjuvant chosen from preserving agents, complexing agents, dyes, alkalinising or acidifying agents, surface-active, anionic, cationic, nonionic, amphoteric agents or their mixtures, anionic, cationic, nonionic or amphoteric polymers as well as their mixtures.

**13.** Process for the cosmetic treatment of hair or the scalp, characterised in that the components (A) and (B) of the combination such as defined in any one of Claims 1 to 12 are applied either by means of a single composition or separately, either simultaneously or successively or spaced out over time.

**14.** Multi-compartment device or "kit", characterised in that it comprises in a first compartment the component (A) such as defined in any one of Claims 1 to 12, and in a second compartment the component (B) such as defined in any one of Claims 1 to 12.

**15.** Combination according to any one of Claims 1 to 12, for its application as medicinal substance in the therapeutic treatment of hair loss.

**16.** Use of the combination according to any one of Claims 1 to 12, for the preparation of a medicinal substance intended for the therapeutic treatment of hair loss.

**Patentansprüche**

**1.** Mischung zum Induzieren und Stimulieren des Haarwuchses und zum Vermindern des Haarausfalls,

dadurch **gekennzeichnet,** daß sie umfaßt:

a) eine Komponente (A), enthaltend in einem physiologisch verträglichen Milieu mindestens ein antibakterielles Mittel, ausgewählt aus Antibiotika der Familie der Makrolide, Pyranoside wie auch der Tetracycline; sowie
b) eine Komponente (B), enthaltend in einem physiologisch verträglichen Milieu mindestens ein Pyrimidinderivat der Formel:

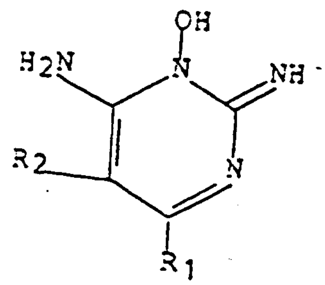

(I)

worin $R_1$ eine

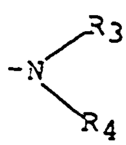

-Gruppe darstellt,

worin $R_3$ und $R_4$ aus Wasserstoff, einer Alkyl-, Alkenyl-, Alkylaryl- oder Cycloalkylgruppe ausgewählt sein können, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-, Hexahydroazepinyl-, Heptamethylenimin-, Octamethylenimin-, Morpholin- und Alkyl-4-piperazidinydylgruppen, wobei die heterozyklischen Gruppen an den Kohlenstoffatomen durch eine bis drei Niedrigalkyl-, Hydroxy- oder Alkoxygruppen substituiert sein können; die Gruppe $R_2$ aus Wasserstoff, einer Alkyl-, einer Alkenyl-, Alkylalkoxy-, Cycloalkyl-, Aryl-, Alkylaryl-, Arylalkyl-, Alkylarylalkyl-, Alkoxyarylalkyl- oder Haloarylalkylgruppe ausgewählt ist, sowie deren physiologisch verträglichen Säureadditionssalze, wobei die Komponenten (A) und (B) einen Teil desselben Mittels bilden oder dazu bestimmt sind, getrennt, sei es gleichzeitig, sei es nacheinander oder nach einem Zeitablauf, auf Kopfhaut oder den Haaren verwendet zu werden.

**2.** Mischung gemäß Anspruch 1,
dadurch **gekennzeichnet,** daß
die antibakteriellen Mittel der Familie der Makrolide aus Erythromycin, Spiramycin, Oleandomycin, Roxytromycin, Josamycin und deren Derivaten und die Pyranoside aus Lincomycin, Clindamycin und deren Derivaten ausgewählt sind.

**3.** Mischung gemäß Anspruch 2,
dadurch **gekennzeichnet,** daß
die Derivate von Erythromycin aus Estolat, Ethylcarbonat, Ethylsuccinat, Glucoheptonat, Lactobionat, Propyionyllaurylsulfat, Propionat, Stearat, Linoleat, den Mono-en-, Bi- oder Tri-en-Estern von Erythromycin, den Estern der Retinoesäure-all-trans und -13-cis von Erythromycin A, wie O-Linoleoyl-2'-erythromycin A, O-Linoleoyl-4''-erythromycin A und O-alpha-Linoleoyl-4''-erythromycin A, die Derivate von Spiramycin aus Spiramycin-di- oder -triacetat ausgewählt sind und daß das Clindamycinderivat das Clindamycinhydrochlorid ist.

**4.** Mischung gemäß eines jeden der Ansprüche 1 bis 3,
dadurch **gekennzeichnet,** daß
die Lincomycinderivate aus Lincomycin und Lincomycinhydrochlorid, die Clindamycinderivate aus Clindamycin und Clindamycinhydrochlorid, -palmitat und -phosphat sowie den Retinoesäurederivaten von Lincomycin und Clindamycin ausgewählt sind.

**5.** Mischung gemäß eines jeden der Ansprüche 1 bis 4,
dadurch **gekennzeichnet,** daß
die Tetracyclinderivate aus Tetracyclin, dem Hydrochlorid, N-(n-Dodecyl)sulfamat, Guajacolglycolat, Guajacolsulfonat, Laurylsulfat, Metaphosphat, dem Tetracyclinphosphatkomplex, Tartratkomplex, Phenoxymethylpenicillinat, Timolsulfonat und dem Thrihydrotrimethoxybenzoat von Tetracyclin ausgewählt sind.

**6.** Mischung gemäß Anspruch 5,
dadurch **gekennzeichnet,** daß
das Pyrimidinderivat Amino-6-dihydro-1,2-hydroxy-1-imino-2-piperidino-4-pyrimidin oder Minoxidil ist.

**7.** Mischung gemäß eines jeden der Ansprüche 1 bis 6,
dadurch **gekennzeichnet,** daß
das antibakterielle Mittel der Familie der Antibiotika in der Komponente (A) in Verhältnissen von 0,01 bis 10 Gew.%, bezogen auf Gesamtgewicht der Komponente (A), insbesondere von 0,1 bis 5 Gew.%, und ganz besonders von 0,3 bis 5 Gew.%, und daß das Pyrimidinderivat der Formel (I) in der Komponente (B) in Verhältnissen von 0,05 bis 10 Gew%, vorzugsweise von 0,1 bis 5 Gew.%, und insbesondere von 0,5 bis 3 Gew.%, vorliegt.

**8.** Mischung gemäß eines jeden der Ansprüche 1 bis 7,
dadurch **gekennzeichnet,** daß
die Mischung als einheitliches Mittel vorliegt, worin der antibakterielle Wirkstoff in Anteilen von 0,1 bis 3 Gew.% und das Pyrimidinderivat der Formel (I) in einem Anteil von 0,5 bis 3 Gew.%, bezogen auf Gesamtgewicht des Mittels, vorliegen.

**9.** Mischung gemäß eines jeden der Ansprüche 1 bis 8,
dadurch **gekennzeichnet,** daß
das physiologisch verträgliche Milieu für die Komponenten (A) und (B) ein Milieu aus Wasser oder einer Mischung aus Wasser und einem oder mehreren organischen Lösungsmitteln oder aus einer Mischung von in der Kosmetik oder Pharmazie verträglichen organischen Lösungsmitteln zusammengesetzt ist.

**10.** Mischung gemäß eines jeden der Ansprüche 1 bis 9,
dadurch **gekennzeichnet,** daß
mindestens eines der Komponenten (A) oder (B) ein Lösungsmittel enthält, ausgewählt aus $C_1$-$C_4$-Niedrigalkoholen, Alkylenglycolen, Alkylethern von Mono- und Dialkylenglycol.

**11.** Mischung gemäß einem der Ansprüche 1 bis 10,
dadurch **gekennzeichnet,** daß
mindestens eine der Komponenten (A) oder (B) durch Verdickungs- oder Geliermittel verdickt ist.

**12.** Mischung gemäß eines jeden der Ansprüche 1 bis 11,
dadurch **gekennzeichnet,** daß
mindestens eine der Komponenten (A) oder (B) auch einen kosmetisch oder pharmazeutisch verträglichen Hilfstoff enthält, ausgewählt aus Konservier-, Komplexier-, Färbungs-, alkalisch- oder sauermachenden, anionischen, kationischen, nicht-ionischen, amphoteren oberflächenaktiven Mitteln oder deren Mischungen, anionischen, kationischen, nicht-ionischen oder amphoteren Polymeren sowie deren

Mischungen.

**13.** Verfahren zur kosmetischen Behandlung von Haaren oder Kopfhaut , dadurch **gekennzeichnet,** daß man die Komponenten (A) und (B) der Mischung gemäß eines jeden der Ansprüche 1 bis 12 aufbringt, und zwar entweder durch ein einheitliches Mittel oder in getrennter Form, sei es gleichzeitig, sei es nacheinander oder nach einem Zeitablauf.

**14.** Zusammenstellung in mehreren Einheiten oder als "Kit", dadurch **gekennzeichnet,** daß sie in einer ersten Einheit die Komponente (A) wie definiert in jedem der Ansprüche 1 bis 12 und in einer zweiten Einheit die Komponente (B) wie definiert in jedem der Anprüche 1 bis 12 umfaßt.

**15.** Mischung gemäß eines jeden der Ansprüche 1 bis 12 zur Anwendung als Medikament bei der therapeutischen Behandlung von Haarausfall.

**16.** Verwendung der Mischung gemäß eines jeden der Ansprüche 1 bis 12 zur Herstellung eines Medikaments zur therapeutischen Behandlung von Haarausfall.